# EUROPEAN PATENT APPLICATION

(11) **EP 3 112 455 A1**
(43) Date of publication of application: **04.01.2017**
(21) Application number: 15755605.1
(22) Date of filing: 15.02.2015
(51) Int. Cl.: C12N 5/10, C12N 15/873

(54) **POLAR BODY GENOME RESTRUCTURED OOCYTES AND PREPARATION METHOD AND USE THEREOF**

(30) Priority: 28.02.2014 CN 201410070925
(71) Applicant: Fudan University, Yangpu, Shanghai 200433 (CN)
(72) Inventor: SHA, Hongying, Shanghai 200433 (CN); ZHU, Jianhong, Shanghai 200433 (CN); CAO, Yunxia, Shanghai 200433 (CN); WANG, Tian, Shanghai 200433 (CN); JI, Dongmei, Shanghai 200433 (CN)
(74) Representative: Keil & Schaafhausen Patent- und Rechtsanwälte PartGmbB
(86) International application number: PCT/CN2015/073099
(87) International publication number: WO 2015/127875

(57) **Abstract**

Disclosed are polar body genome restructured oocytes, the preparation method thereof and the use thereof in preparing the materials for preventing the occurrence of mitochondrial material genetic diseases.

## Description

### BACKGROUND OF THE INVENTION

### 1. Field of the Invention

This invention belongs to biomedical field, it relates to polar body genome and its preparation method, specifically, it relates to polar body genome restructured oocytes , as well as the preparation method and preparation method, especially, the preparation method in preparing the materials for preventing occurrence of mitochondria maternal genetic diseases.

### 2. Description of the Prior Art

According to the study, the mitochondria DNA is another genome independent of cell nucleus chromosome, it is called No. 25 chromosome of human beings, and its genetic characteristics are non-mendelian inheritance, which are also called as maternal inheritance, that is, the mother transmits the mtDNA to her son and daughter, but only the daughter can transmit the mtDNA to next generation.

The mitochondriopathy is a kind of multisystem disease caused by that the genetic defects cause defects of the mitochondria metabolic enzymes, which may bring ATP composition obstacle and insufficient energy. The mtDNA mutation in the oocytes may cause maternal-line familial diseases, that is, maternal mitochondria diseases. According to the incomplete statistics, among the patients with myasthenia symptoms caused by mitochondria diseases, there will be one patient (among 200 patients) may inherit mitochondria myasthenia to the next generation. On the other hand, important organs of human body are more easily affected by the mutations (the parts that have high energy demands, such as brain, heart, kidney, skeletal muscle and internal secretion gland etc).

The research shows that most of mitochondria diseases can affect normal functions of multiple organ systems. The clinical phenotypes of such diseases are very extensive, including mitochondria brain myopathy, mitochondria myopathy, gastrointestinal syndrome, myodystony, diabetes mellitus, cardiomyopathy, alzheimer disease, Parkinson's disease, Huntington's chorea, stupid, and deaf etc. At present, in clinical, there aren't effective therapy methods for the diverse mitochondria diseases, and the patients can only relieve the symptoms for short time relying on medicines, their living quality can't be guaranteed. At the same time, the mitochondria DNA are transmitted to the next generation by oocytes cytoplasm and maternal genetic, therefore, the patients shall not produce, or it may bring suffering and misfortune for the family.

The development of oocytes/ embryo micromanipulation technology (nuclear transfer) brings hope for therapy of mitochondria diseases. The mitochondria genetic substances adopt maternal mode of inheritance, that is, they are transmitted to the next generation by the mother, therefore, the oocytes/ embryo micromanipulation technology can be used for mitochondria replacement (MR) to the oocytes of the mother with mitochondria diseases, so that she can get normal mitochondria, mitochondria functions of the descendants generated with the oocytes after being replaced will be improved or completely returned to normal.

At present, MR technology includes, in the metaphase, spinlde-chromosome transfer (ST), to transfer genetic substances in oocytes of the patient to cytoplasm of the healthy oocytes that the genetic substances have been removed; As well as pronucleus transfer (PNT), transfer germ cell's pronucleus of husband and wife with the patient to cytoplasm of the healthy germ cell that the pronucleus has been removed, and so on. In recent years, someone has got the descendants after mitochondria replacement using two MR technologies above in animal experiments (the mouse, primate).

Therefore, since 2011, Human Fertilisation and Embryology Authority of the United Kingdom began to summarize the research results of mitochondria replacement in recent years, brew corresponding policies, some trends drive people to conduct further clinical and trial study. At the same time, the institution also carried out evaluation for safety of using people-people mitochondria replacement to reconstruct oocytes, and they believed that such technology doesn't change main genetic substance of the oocytes-nucleus DNA, and which is different with the preparation method of clones, but no evident safety hazard is found. Purportedly, the institution will issue the policies corresponding to people's mitochondria replacement after extensively listening to public opinions (Human Fertilisation and Embryology Authority mitochondria public consultation 2012: http://www.hfea.gov.uk/6896.html).

Although HFEA has evaluated the safety of mitochondria replacement technology above, and they believed that it is safe, but the bottleneck effect of mitochondria DNA inheritance makes the technology above can't thoroughly prevent occurrence of mitochondria maternal genetic diseases. Because there are about 0.1 million mitochondria DNAs in each oocytes cell of human body, but only a small random number (2∼200 pieces) can become mature oocytes cells and transferred to descendant, the process that, in forming period of oocytes cells, the number of mitochondria DNAs is sharply reduced is called as "genetic bottleneck effect" in the industry.

The descendant mitochondria DNA's population types can be formed through replication and augmentation of the "bottleneck" mitochondria DNAs. Therefore, in order to prevent mitochondria diseases from entailing to the descendant as far as possible, we must ensure that, after the replacement, there aren't the patient's mitochondria DNA in the oocytes reconstructed. While, the research results show that either metaphase chromosome or pronucleus, because of energy demands, their peripheries are a large number of mitochondria, which accounts for about 40% of gross mitochondria, in the process of nucleus replacement, such mitochondria enters new oocytes cytoplasm with the nucleus, so that the reconstructed oocytes after being replaced still contains a certain number of mutation mtDNAs, and these mtDNAs may be replicated and augmented in the descendant through the "genetic bottleneck ", when the accumulation reaches certain threshold values, they still may cause diseases of the descendant.

According to the problems above, inventor of the invention proposes to provide a polar body genome to reconstruct the oocytes, as well as the preparation method and preparation method, especially the preparation method in preparing the materials for preventing occurrence of mitochondria maternal genetic diseases. There aren't the patient's mitochondria DNA in the oocytes reconstructed with this invention, which will be able to thoroughly prevent occurrence of mitochondria maternal genetic diseases.

### 3. BRIEF SUMMARY OF THE INVENTION

Preparation method of the invention is to overcome the defects and insufficiency of existing technology, and provide a method for reconstructing oocytes with polar body genome and the preparing method.

Preparation method of the invention is to provide the preparation method of the reconstructed oocytes mentioned in preparing the materials for preventing occurrence of mitochondria maternal genetic diseases. The reconstructed oocytes produced with this invention doesn't contain mitochondria DNA, thus can effectively interdict the related diseases related to the cytoplasm, especially, the invention can be used for mitochondria replacement to reach the effects of treating mitochondria maternal genetic diseases, actively, and thoroughly prevent occurrence of mitochondria maternal genetic diseases.

Theoretic basis of this invention: Forming process of the female sex cell will experience meiosis twice to form a large haploid, that is, the oocytes and 2∼3 small cells, such small cells are called as polar, a large secondary oocyte and a small first polar body (PB1) are formed in period of the first meiosis; similarly, a small second polar body (PB2) is formed in period of the second meiosis, either PB1 or PB2, they have the following characteristics: (1) Although volume of the polar is far smaller than the oocytes, it can obtain same genetic substances with the oocytes; (2) There is little cytoplasm (mitochondria) in the polar, therefore, when it is used for providing genome to reconstruct the oocytes, there will be little or no disease mitochondria is taken to the oocytes reconstructed.

The invention adopts two characteristics of the polar: It contains same female DNA with that in the oocytes cytoplasm; at the same time, it carries little or no cytoplasm substance (mitochondria), to provide a kind of reconstructed oocytes with the polar body genome, and its application in material preparation for preventing maternal mitochondria genetic diseases.

Preparation method of this invention will be achieved through the following technical solution.

Construct a reconstructed oocytes with the genome provided by the first and/or the second polar of the patient's oocytes and germ cell and the healthy people's oocytes, the reconstructed oocytes only contain mitochondria of the healthy people's oocytes.

In this invention, the first polar mentioned comes from meiosis metaphase oocytes of the female patient with mitochondria diseases.

The second polar mentioned comes from the germ cell combined meiosis metaphase oocytes of the female patient with mitochondria diseases and her spouse's sperm.

This invention adopts micromanipulation methods with isolation, to immigrate the first and/or second polar of the patient's oocytes and germ cell to cytoplasm of the healthy people's oocytes or germ cell that the female genetic substances have been removed; the steps include:
(1) reconstructing the oocytes with the first polar body genome of the female patient with mitochondria diseases and oocytes cytoplasm of the healthy female;
(2) using the second polar body genome of the female patient with mitochondria diseases to replace female pronucleus of the germ cell of the healthy female and the patient spouse for reconstructing the embryo;
(3) inspecting contents of the mitochondria from two sources in the embryos developed from the reconstructed oocytes/ reconstructed germ cell; (Heterogeneity of the mitochondria)
(4) the animal model test can be used to verify the interdicting effects of the three kinds of genetic substances (father and mother nucleus DNA and the mitochondria DNA provided by healthy people) contained in the embryo obtained to maternal inheritance of mitochondria diseases.

More concretely, the core technical solution in this invention is using isolation method to prepare the reconstructed oocytes of mitochondria replacement and reconstruct embryo, include:
(1) Using the first polar body genome of the female patient with mitochondria diseases and oocytes cytoplasm of healthy female to reconstruct the oocytes.
   Using the first polar genetic substance ovulated from the oocytes of the female patient with mitochondria diseases to replace the second phase of genetic substance in cytoplasm of the oocytes, and reconstruct a oocytes with oocytes cytoplasm (the genetic substance has been removed) of healthy female; such oocytes reconstructed has same fertilization and development ability with normal oocytes, and the descendant obtained can thoroughly prevent occurrence of mitochondria diseases.
   Put oocytes of the patient mentioned and the healthy female's oocytes into the G-gamete operation fluid including CB in the microscopy working room, 10 minutes later, use a oocytes-holding needle to hold a oocytes of the patient (containing living and full polar) and place it at the position of 12 o'clock. Use an 8µm denucleation needle controlled by Piezo pulse to absorb PB1, when the plasmalemma is simply broken in 7% of PVP, it is injected into oocytes of the healthy female that is denucleated, when the reconstructed oocytes is sufficiently washed in HTF, it is moved to HTF for in vitro fertilization.
   Take the spouse's sperm of the patient and place it to HTF to obtain energy for an hour, and then add moderate sperm to liquid drop of the reconstructed oocytes for in vitro fertilization. When the fertilization is completed for 6 hours, dual prokaryotic germ cell will be picked out and moved to G1 nutrient solution for 72 hours, to observe the embryonic development under the microscope every day, according to the clinical requirements, the embryo can be further transferred to G2 to blastaea stage, and the embryo at various stages can be used for implanting or cryopreservation.
   The reconstruction mentioned in above preparing methods refers to the nuclear transfer technique mediated with the micromanipulation is used to form reconstructed oocytes/germ cell with the patient's genetic substance and cytoplasm of the healthy oocytes.
(2) Using the second polar body genome of the female patient with mitochondria diseases to replace pronucleus of the germ cell of the healthy female and her patient spouse for reconstructing an embryo.
   Put the patient and healthy female and the germ cell of patient spouse into the g-gamete containing CB in the microscope working room, 10 minutes later, use a oocytes-holding needle to hold a germ cell of the patien and place it at the position of 12 o'clock. Use an 8µm denucleation needle controlled by Piezo pulse to absorb PB2 (pay attention to keep completeness of the plasmalemma), and then use a 15µm bevel connection denucleation needle to transfer PB2 into inactivated sendai virus to treat for several seconds, and then transfer it to the transparent belt of the healthy feamale's germ cell that female pronucleus has been removed, so that it is closely contact with the oocytes plasmalemma to promote integration of the both, after integration, the reconstructed zygote is thoroughly washed with G1 liquid drop and then transferred to G1 for cultivating, the embryo can be further transferred to G2 for cultivating to blastaea stage, and the embryo at various stages can be used for implanting or cryopreservation.
(3) Inspect contents of the mitochondria from two sources in the embryos developed from the reconstructed oocytes/ reconstructed germ cell (heterogeneity of the mitochondria).
   Split embryos at various stages, use a whole genome kit (TIANGEN, BEIJING) to extract DNA, and then, according to the mitochondria DNA series of the patient and the donating female of healthy oocytes, select a section of series where the different DNA locus is, and then design primers to increase the products of the section of series on mitochondria of the both. According to the different DNA locus, we can design marking primers to increase the second round of products for pyrosequencing.
   For content inspection of descendant mitochondria, the pyrosequencing shall be used.
(4) The animal model test can be used to verify the interdicting effects of the three kinds of genetic substances (father and mother nucleus DNAs, as well as the healthy supplier's mitochondria DNA) contained in the embryo obtained to maternal inheritance of mitochondria diseases.

Use to kinds of mouse models containing different mitochondria to prepare reconstructed oocytes above, and transplant it to in vivo of the pseudopregnancy mother mouse to produce the mitochondria replacement mouse of polar body genome transplant, at the same time, to prepare mitochondria replacement mouse under the conditions of existing technology (spindle body, pronucleus replacement), and verify the interdicting effects of the reconstructed oocytes obtained with this invention to maternal inheritance of mitochondria diseases.

The polar body genome restructured oocytes in this invention can be used to further prepare the materials for preventing occurrence of mitochondria maternal genetic diseases.

In this invention, the oocytes reconstructed with the polar body genome has the following advantages:
(1) There is little mitochondria (PB2) in the polar, even that the mitochondria (PB1) is in deficiency completely, the new therapy path and means have been developed for actively and thoroughly preventing occurrence of mitochondria maternal genetic diseases;
(2) The polar body genome is wrapped with the plasmalemma, and its volume is small, easy to operate, the plasmalemma can be used to ensure that in process of the operation, the genome isn't lost;
(3) The oocytes reconstructed in this invention has the ability of in vitro fertilization, cleavage and development that normal oocytes has;
(4) The germ cell reconstructed in this invention has the ability of cleavage and development that normal germ cell has;
(5) In this invention, all descendants come from the first polar only have the mitochondria (the mitochondria of healthy female) with cytoplasm donor, no nucleus donor (that is the mitochondria of the patient with mitochondria diseases) is detected, that is, the completely healthy descendant coming from the patient with mitochondria diseases is obtained in this invention;
(6) The pyrosequencing methods established in this invention have high sensitivity in detecting heterogeneity and specificity of the mitochondria.

In this invention, the polar body genome restructured oocytes can be used for mitochondria replacement, to reach the effects of treating maternal mitochondria diseases; there aren't mitochondria DNA of the patient in the oocytes reconstructed in this invention, thus can effectively interdict the occurrence of diseases related to the cytoplasm, to actively and thoroughly prevent occurrence of maternal mitochondria genetic diseases.

### 4. BRIEF DESCRIPTION OF THE DRAWINGS

FIG. 1 is technical route of this invention shows that: Donor oocyte / zygote (comes from the patient with female mitochondria diseases); Recipient oocyte / zygote (comes from the healthy female); first polar body transfer; Spindle transfer; second polar body transfer; pronucleus transfer; mitochondria replacement; undetectable donor mtDNA carry-over; low to medium donor mtDNA carry-over; very low donor mtDNA carry-over; medium to high donor mtDNA carry-over.
FIG. 2 shows mitochondria distribution around PB1, Spindle, PB2 and pronuclei, of which,
   A: The mitochondria distribution in cytoplasm of the oocytes and the first polar;
   B: The mitochondria distribution in cytoplasm of the germ cell and the second polar;
   C: The mitochondria carried around the first polar, the compound of spindle body-chromosome, the mitochondria carried around the second polar and pronucleus; D: Fluorescence intensity analysis of the mitochondria around the first polar, compound of spindle body- chromosome, the second polar, and pronucleus. MitoTrackor: Fluorescence probe of the mitochondria; Hochest: Nucleus fluorescence probe.

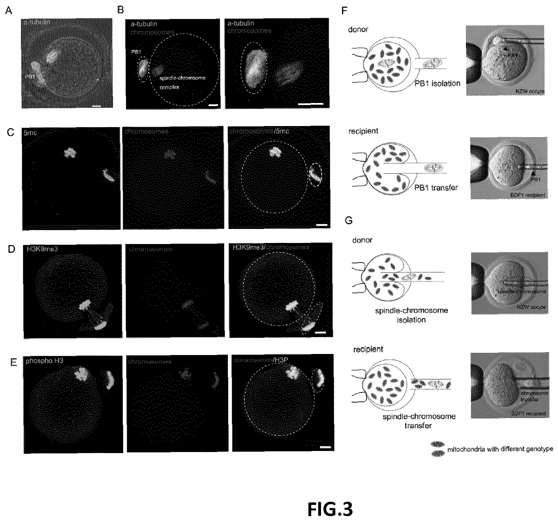

FIG. 3 shows that the epigenetic characteristics of PB1 and spindle-chromosome are consistent, PB1 can be used to replace spindle-chromosome for treating maternal mitochondria diseases, of which,
   A, B: α-tubulin dyeing shows that the form characteristics of PB1 and spindle-chromosome are consistent; C, D, E: The antibody dyeing of 5-methylcystein methylation, 3- methylation of histone, and 3- phosphorylation of histone shows that epigenetic characteristic of PB1 and spindle-chromosome are consistent; F: The replacement mode and entity map of the first polar; G: The replacement mode and entity map of the spindle body.

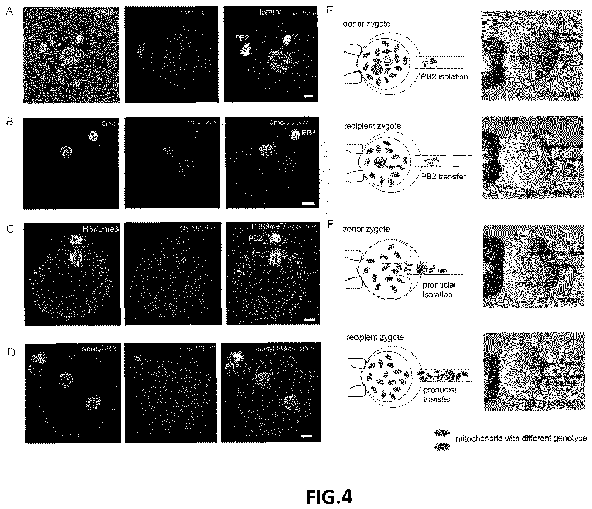

FIG. 4 shows that epigenetic characteristics of PB2 and female pronucleus are consistent; PB2 can be used to replace the female pronucleus for treating maternal mitochondria diseases, of which,
   A, B: lamin B1 dyeing shows that the form characteristics of PB2 and female pronucleus are consistent; C, D, E: The antibody dyeing of 5-methylcystein methylation, 3- methylation of histone, and 3- acetylation of histone shows that epigenetic characteristics of PB2 and the female pronucleus are consistent; F: The replacement mode and entity map of the second polar; G: The replacement mode and entity map of the pronucleus replacement.

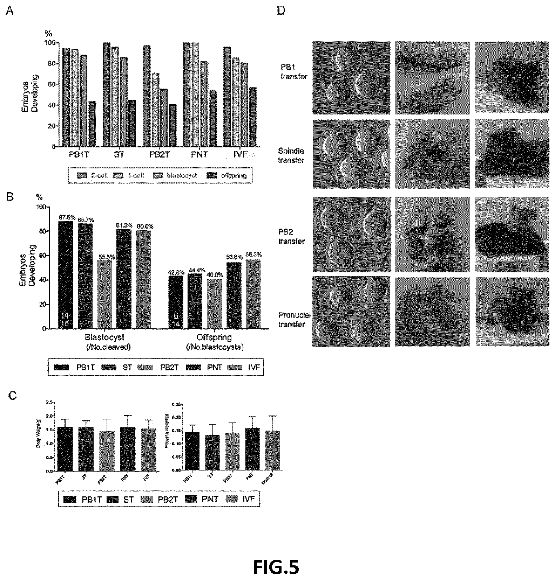

FIG. 5 shows four kinds of mitochondria replacement mouse generated by PB1T, ST, PB2T, PNT, and the efficiency, of which,
   A, B: Efficiency of the four kinds of mitochondria replacement technologies (PB1T, ST, PB2T, and PNT), IVF is contrast of the four technologies; C: Birth weights and placenta weights of the mouse replaced with the four kinds of mitochondria (PB1T, ST, PB2T, and PNT) have no significant difference with those of IVF mouse; D: The embryo, newborn, and adult individual replaced with the four kinds of mitochondria (PB1T, ST, PB2T, and PNT).

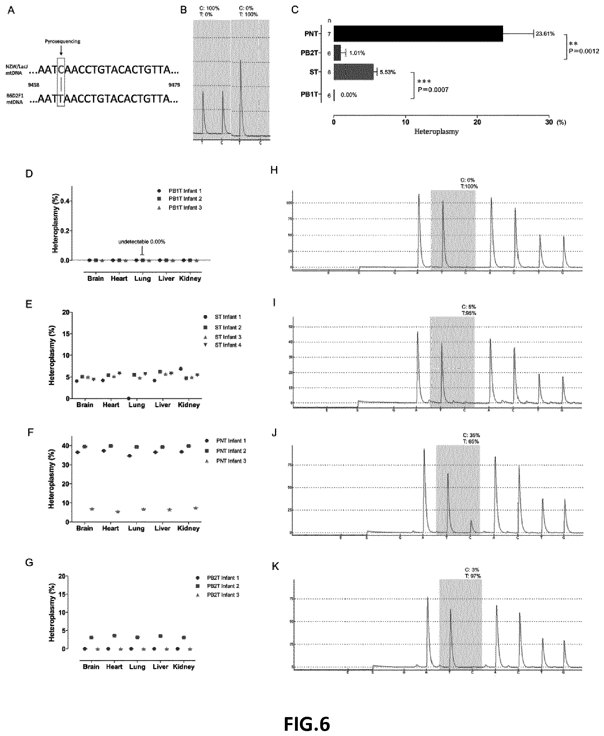

FIG. 6 shows the mitochondria heterogeneity of the nucleus donor and cytoplasm donor in vivo of the mouse replaced with the four kinds of mitochondria (PB1T, ST, PB2T, and PNT), of which,
   A: The detected mitochondria locus of the nucleus donor (NZW mouse) and cytoplasm donor (BDF1 mouse); B: Indicates the pyrosequencing FIG. when C and T are 100%; C: The comparison of mitochondria heterogeneity of the nucleus donor and cytoplasm donor in vivo of the mouse replaced with the four kinds of mitochondria (PB1T, ST, PB2T, and PNT); D, E, F, G: Successively refer to mitochondria heterogeneity's distributions of the nucleus donor and cytoplasm donor in the important organs of brain, heart, lung, liver and kidney etc in vivo of the mouse replaced with the four kinds of mitochondria (PB1T, ST, PB2T, and PNT); H, I, J, K: Successively refer to mitochondria heterogeneity's representative atlas of the nucleus donor and cytoplasm donor in the important organs of brain, heart, lung, liver and kidney etc in vivo of the mouse replaced with the four kinds of mitochondria (PB1T, ST, PB2T, and PNT).

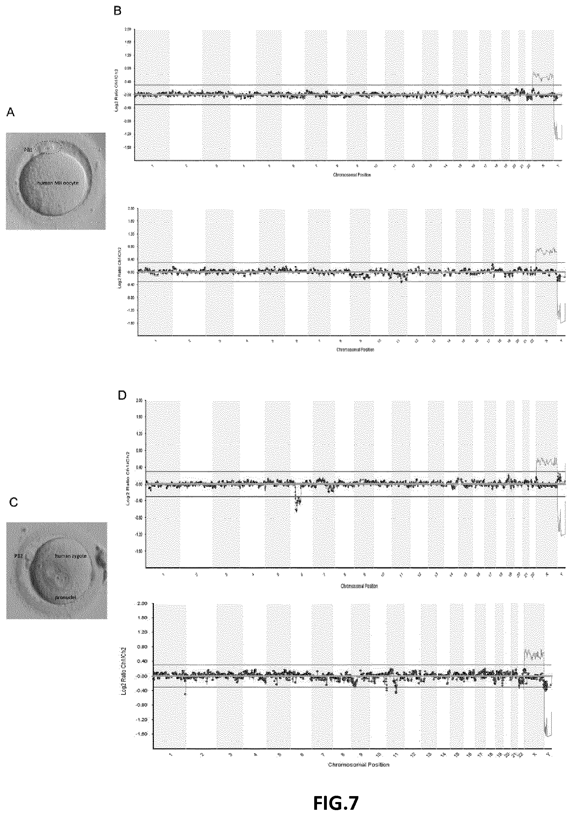

FIG. 7 shows that the genetic characteristics of PB1 and spindle-chromosome, germ cell PB2 and Female Pronucleus are consistent, of which,
   A: Oocytes and the first polar of human beings; B: The comparative genomics detection shows that the genetic characteristics of the oocytes's first polar and the nucleus chromosome in its cytoplasm are consistent; C: Germ cell and the second polar of human beings; D: The comparative genomics detection shows that the genetic characteristics of the germ cell's second polar and the pronucleus in its cytoplasm are consistent.

### 5. DETAILED DESCRIPTION OF THE PREFERRED EMBODIMENTS

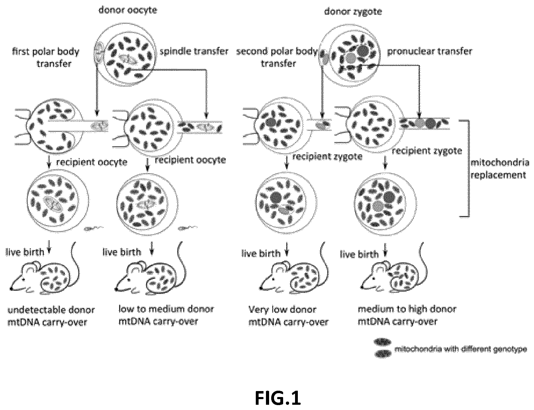

We will further set forth this invention combining with concrete implementation of animal model below, and compare it with existing mitochondria replacement (ST, PNT) (FIG. 1: Research diagram of this invention). And we shall understand that these implementation cases can only be used to explain this invention, not to be used for limiting the invention.

### I . Research Materials and Methods

### (I) Research Materials

New Zealand white mouse, in this invention, it is used to simulate the patient with female mitochondria diseases. The hybrid generation mouse of BDF1 of C57BL / 6 and DBA2, in this research, is used to simulate the healthy female who donates oocytes cytoplasm. While the ICR mouse is treated as surrogacy mother in this invention.

### (II) Research Methods

### 1. The feasibility analysis that PB1 and PB2 are used for treating maternal mitochondria diseases

### (1) Comparison of the mitochondria carried by PB1, spindle, PB2 and Pronuclei

Use the mitochondria fluorescence probe of MitoTrackor (250nM) to dye the oocytes and germ cell for 10 minutes, use Hochest33342 re-dye the nucleus for 10 minutes. Put the dyed oocytes embryo in CB operation liquid drop of 5µg/ ml, use a 15µm enucleation needle to take the PB1, Spindle, PB2 and Pronuclei out, and then transfer them into the liquid drop of the culture dish cabinet specially used for the confocal microscopy, and then observe the distributions under the confocal microscopy.

### (2) Comparison of the epigenetic characteristics of PB1 and spindle, PB2 and Pronulcei.

The fresh oocytes and germ cell are fixed and conducted permeabilizing and sealing treatment with 4% of paraformaldehyde, and then, they shall be conducted epigenetic inherent immunohistochemical detection, the primary antibodies detected includes: Anti-tubulin (1/200, Sigma, T8203), anti-lamin B1(1:50, Abcam, ab8982), anti-H3K9me3 (1/200, Abcam, ab8898), anti-H3P (1/100, Abcam, ab7031), anti-acetyl H3 (1:250, Millipore 06599), and 5mC antibody (1:200, Abcam, ab10805).

All primary antibodies are incubated in 4C over the night. On the next day, use PBS to sufficiently wash the oocytes and germ cell, and then, the different primary antibodies respectively are marked with the second fluorescence antibody of DyLight 488, IgG (1:500, Jackson) or Alexa488, and IgG (1:500, Invitrogen) (incubated for 1.5h, at 37°C). After the second antibody marking is over, use PI to re-dye the nucleus. Put the dye oocytes and germ cell into the liquid drop of the culture dish cabinet specially used for the confocal microscopy, and then observe the epigenetic similarities and differences under the confocal microscopy.

### 2. Prepartions of the four kinds of mitochondria (PB1T, ST, PB2T, and PNT) mice and their descendant reproduction

### (1) Preparations of PB1T and ST mice

Inject 5IU PMSG to abdominal cavities of the 6-8 weeks of NZW, BDF1 female mice, and then, re-inject 5IU HCG to abdominal cavities of them at an interval of 48h. 13hours later after HCG injection, take mature oocyte wrapped with cumulus cell from expansion part of the fallopian tube, and place it into the G-gamete operation liquid containing 0.1% hyaluronidase at the temperature of 37°C in the incubater where 5%CO2 is properly saturated, until the cumulus cell becomes loose (2∼3min). The oocytes after degranulation shall be placed into an incubator for recovering for 30min.

Place the oocytes cells of NZW and BDF1 into the G-gamete operation liquid containing CB in the microscopy room, 10 minutes later, use an oocytes-holding needle to hold a NZW oocytes (including living and saturated polar), and make the polar at the position of 12 O'clock. Use a 8µm denucleation needle controlled with Piezo pulse to absorb PB1 to simply break the plasmalemma in 7% of PVP, and then inject it to BDF1 denucleated oocytes. The final reconstructed shall be placed into HTF for sufficiently washing and transferred to HTF for in vitro fertilization.

Use a 8µm denucleation needle controlled with Piezo pulse to slightly absorb the compound of spindle body- chromosome of NZW oocytes together with a little of cytoplasm (for short, karoplast), then use a 15µm bevel connection denucleation needle to transfer karoplast into inactivated sendai virus to treat for several seconds, and then transfer it to the transparent belt of denucleated BDF1 oocytes, so that it is closely contact with the oocytes plasmalemma of BDF1 to promote integration of the both, after integration, the reconstructed zygote is thoroughly washed with HTF liquid drop and then transferred to HTF in vitro fertilization.

The 12 weeks of male mouse died for taking-off of the cervical spine, take the sperm from the epididymis and place it into HTF to absorb energy for an hour, and then add moderate sperm to liquid drop of the reconstructed oocytes for in vitro fertilization. When the fertilization is completed for 6 hours, dual prokaryotic germ cell will be picked out and moved to G1 nutrient solution for 72 hours, to observe the embryonic development under the microscope every day, 72 hours later, it will develop to reconstructed embryo of the blastaea and transferred to in vivo of a pseudopregnancy mother mouse for farrowing. 16 days later, conduct caesarean to the pregnant mother mouse, the viable newborn mouse will be cultivated.

### (2) Preparations of PB2T and PNT mice

Inject 5IU PMSG to abdominal cavities of the 6-8 weeks of NZW, BDF1 (The injection PMSG time of NZW mouse shall be about 2hours earlier than BDF) female mice, and then, re-inject 5IU HCG to abdominal cavities of them at an interval of 48h, let it copulate with a 12-week male mouse in the cage. 18hours later after HCG injection, recycle the zygote wrapped with cumulus cell from expansion part of the fallopian tube, and place it into the G-gamete operation liquid containing 0.1% hyaluronidase at the temperature of 37°C in the incubater where 5%CO2 is properly saturated, until the cumulus cell becomes loose (2∼3min). The oocytes after degranulation shall be placed into an incubator for recovering for 30min.

Place the zygote of NZW and BDF1 into the G-gamete operation liquid containing CB in the microscopy room, 10 minutes later, use an oocytes-holding needle to hold a NZW zygote, and make the polar at the position of 12 O'clock. Use a 8µm denucleation needle controlled with Piezo pulse to absorb PB2 (pay attention to keep completeness of the plasmalemma), and then use a 15µm bevel connection denucleation needle to transfer PB2 into inactivated sendai virus to treat for several seconds, and then transfer it to the transparent belt of the BDF1 oocytes that female pronucleus has been removed, so that it is closely contact with the BDF1 oocytes plasmalemma to promote integration of the both, after integration, the reconstructed zygote is thoroughly washed with G1 liquid drop and then transferred to G1 for cultivating.

Use an oocytes-holding needle to remove the NZW zygote of the second polar, and use a 15µm bevel connection denucleation needle to slightly absorb the male & female dual pronucleus, and transfer it into inactivated sendai virus to treat for several seconds, and then transfer it to the transparent belt of the BDF1 oocytes that male & female dual pronucleus has been removed, so that it is closely contact with the oocytes plasmalemma of BDF1 to promote integration of the both, after integration, the reconstructed zygote is thoroughly washed with G1 liquid drop and then transferred to G1 for cultivating.

Observe the embryonic development under the microscope every day. 72 hours later, it will develop to reconstructed embryo of the blastaea and transferred to in vivo of a pseudopregnancy mother mouse in estrus synchronization for farrowing. 16 days later, conduct caesarean to the pregnant mother mouse, the viable newborn mouse will be cultivated.

### 3. Heterogeneity comparison for the four mitochondria (PB1T, ST, PB2T and PNT) replacement mice (♀) and their descendants

Take mice tails from the four mitochondria replacement mice and their descendants, use a whole genome kit (TIANGEN, BEIJING) to extract DNA, and design the primers of 5'- atggctactggattccatgg-3'and 3'- gctcctatgaagcttcatgg-5' to increase 9201∼11102 series of products on the mitochondria. Compound biotin labeling marking primers of TTTGAAGCCGCAGCATGA (forward), ATTTATTTGGGGGAGTCAGAATGC (reverse-biotin) to increase the second round of products for pyrosequencing. And the pyrosequencing primer is GAATAAACCCAGAAGAGAGT, operation of pyrosequencing shall be executed according to operating instructions for the instruments.

### 4. Comparison of the genetic characteristics of PB1 and spindle, germ cell PB2 and Pronulcei

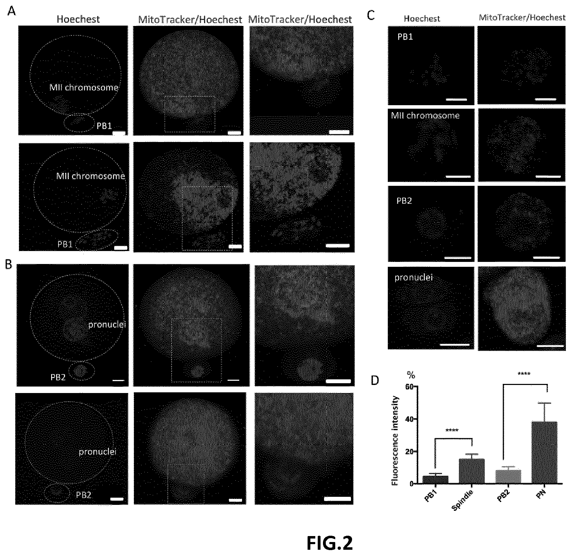

Place the fresh oocytes and germ cell into the operation liquid drop containing 5µg / ml CB, then use a 15µm denucleation needle to take PB1, Spindle, PB2 and Pronuclei out ,and transfer them into a PCR tube, use a use a whole genome kit to conduct single-cell increase, and then conduct comparative genomics chip detection (cGH) to the increased products, and compare whether the genetic characteristics of PB1 and spindle , PB2 and Pronulcei are consistent.
The results show that:
1. PB1 and PB2 have the basis and advantage (relative to ST and PNT) that can be used for treating maternal mitochondria diseases.
   (1) Relative to spindle and pronuclei, PB1 and PB2 carry few mitochondria
      Use a MitoTrackor fluorescence probe to mark the dyeing (FIG. 2), the results show that PB1 and PB2 carry little mitochondria, especially, and almost no mitochondria can be detected in PB1; while the corresponding part-Spindle is surrounded with plenty of mitochondria, because movement of Spindle needs a lot of energy; the corresponding part of PB2-Pronuclei is surrounded with more mitochondria, because of that after the oocytes being fertilized, the mitochondria will be sharply increased, at the same time, the female and male pronucleuses continuously close to prepare splitting, this process needs a lot of mitochondria to provide energy. Therefore, use PB1 to replace ST; use PB2 to replace PNT, the quantity of disease mitochondria carried to the reconstructed oocytes/embryo will be significantly reduced.
   (2) Epigenetic inheritance of PB1 and spindle-chromosome, PB2 and Female Pronucleus is consistent
      The immumohistochemical staining dyeing analysis results show that the epigenetic inheritance characteristics of PB1 and spindle-chromosome are consistent, expressions of the epigenetic biomarkers of H3K9me3, H3P and 5mC antibody can be detected in PB1 and spindle-chromosome (FIG. 3); the genetic characteristics of PB2 and Female Pronucleus are consistent, expressions of H3K9me3, acetyl H3, and 5mC can be detected in PB2 and Female Pronucleus (FIG. 4).
2. The efficiency that PB1 and PB2 provide genomes to birth mitochondria replacement descendants hasn't significant difference with that of ST, PNT and IVF embryo
   In this invention, we have used mitochondria replacement research with a mouse as the model, and got reconstructed mice of PB1 and PB2, comparing with existing mitochondria replacement technology ST, PNT and conventional IVF, the acquisition efficiency hasn't significant difference, and comparing with ST, PNT and conventional IVF, the descendant's body weight and placenta weight also haven't significant difference (see FIG. 5).
3. PB1T mitochondria replacement mouse only contains the mitochondria of cytoplasm donor, that is, the healthy oocytes cytoplasm donor
   The pyrosequencing results show that PB1T mitochondria replacement mouse only contains the mitochondria of cytoplasm donor, without the mitochondria of genome donor, that is, heterogeneity of the mouse is 0%, and it is significantly less than heterogeneity of the ST mitochondria replacement mouse (5%); PB2T mitochondria replacement mouse contains few genome donor, that is, the mitochondria comes from the patient (1%), the value is significantly lower than heterogeneity of PNT mitochondria replacement mouse (25%).
4. The genetic characteristics of PB1 and spindle-chromosome, as well as PB2 and Female Pronucleus are consistent
   Using comparative genomics chip to detect oocytes /germ cell of human beings, the result show that the genetic characteristics of PB1 and spindle-chromosome, PB2 and Female Pronucleus are consistent, with 23 chromosomes, no DNA breakage or increase (FIG. 7), which indicates the feasibility that the polar body genome restructured oocytes in this invention can be used for mitochondria replacement to treat maternal mitochondria genetic diseases.

## Claims

1. The polar body genome restructured oocytes, it is consisted of the genome provided by the first and/or the second polar of the patient's oocytes and germ cell, as well as cytoplasm of healthy people's oocytes, the oocytes reconstructed only contains mitochondria of the healthy people's oocytes.

2. The polar body genome restructured oocytes as claimed in claim 1, wherein the first polar mentioned comes from the oocytes in meiosis metaphase of the female patient with mitochondria diseases.

3. The polar body genome restructured oocytes as claimed in claim 1, wherein the second polar mentioned comes from germ cell combined the oocytes in meiosis metaphase of the female patient with mitochondria diseases and the spouse's sperm.

4. A preparation method of the polar body genome restructured oocytes as claimed in claim 1, wherein it adopts micrurgical technique to immigrate the first and/or second polar of the patient's oocytes and germ cell to cytoplasm of the healthy people's oocytes or germ cell that the female genetic substances have been removed; the steps include:
(1) reconstructing the oocytes with the first polar body genome of the female patient with mitochondria diseases and oocytes cytoplasm of the healthy female;
(2) using the second polar body genome of the female patient with mitochondria diseases to replace female pronucleus of the germ cell of the healthy female and the patient spouse for reconstructing the embryo;
(3) inspecting contents of the mitochondria from two sources in the embryos developed from the reconstructed oocytes/ reconstructed germ cell;
(4) the animal model test can be used to verify the interdicting effects of the three kinds of genetic substances contained in the embryo obtained to maternal inheritance of mitochondria diseases.

5. The preparation method as claimed in claim 4, wherein three genetic substances mentioned are father, mother nucleus DNA and the mitochondria DNA of healthy supplier.

6. The preparation method as claimed in claim 4, wherein content inspection of the mitochondria mentioned adopts pyrosequencing method.

7. The polar body genome restructured oocytes and the use thereof in preparing the materials for preventing the occurrence of mitochondrial material genetic diseases.
